(19) Europäisches Patentamt
European Patent Office
Office européen des brevets

(11) Numéro de publication: **0 504 005 B1**

(12) **FASCICULE DE BREVET EUROPEEN**

(45) Date de publication de fascicule du brevet: **03.05.95**

(51) Int. Cl.[6]: **A61K 7/13**, A61K 7/06

(21) Numéro de dépôt: **92400553.1**

(22) Date de dépôt: **03.03.92**

(54) **Composition pour la coloration enzymatique des fibres kératiniques, notamment des cheveux, et son application dans un procédé de coloration.**

(30) Priorité: **08.03.91 FR 9102858**

(43) Date de publication de la demande:
**16.09.92 Bulletin 92/38**

(45) Mention de la délivrance du brevet:
**03.05.95 Bulletin 95/18**

(84) Etats contractants désignés:
**AT BE CH DE DK ES FR GB GR IT LI LU NL PT SE**

(56) Documents cités:
**FR-A- 2 112 549**
**US-A- 2 539 202**
**US-A- 3 251 742**
**US-A- 3 957 424**

**BROCKS (1967, Phytochemistry, 6, 777-783)**

(73) Titulaire: **PERMA Société Anonyme**
**29bis, rue d'Astorg**
**F-75384 Paris Cédex 08 (FR)**

(72) Inventeur: **Roure, Myriam**
**9, rue d'Oseille**
**F-51100 Reims (FR)**
Inventeur: **Delattre, Paul**
**88, rue de l'Egalité**
**F-59700 Marcq en Baroeul (FR)**
Inventeur: **Froger, Hubert**
**290, rue de Charenton**
**F-75012 Paris (FR)**

(74) Mandataire: **Phélip, Bruno**
**c/o Cabinet Harlé & Phélip**
**21, rue de La Rochefoucauld**
**F-75009 Paris (FR)**

EP 0 504 005 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition pour la coloration des fibres kératiniques, notamment des cheveux , comprenant un enzyme ,de préférence une laccase . Elle est également relative à un procédé de coloration des cheveux utilisant ladite composition.

Actuellement, à la connaissance de la demanderesse, la seule technique de coloration capillaire capable de couvrir correctement et de façon durable les cheveux est la technique de coloration d'oxydation.

Au cours de la réaction d'oxydation, des précurseurs de colorants , qui sont des composés aromatiques appartenant aux familles des diamines, aminophénols (ou aminonaphtols) et des phénols (ou naphtols) , sont oxydés en présence de péroxyde d'hydrogène et d'ammoniaque.

Dans une première étape, ces précurseurs se transforment en radicaux intermédiaires très réactifs qui se couplent entre eux pour former, au cours de la deuxième étape de condensation oxydative, des polymères colorés pouvant se fixer sur la kératine.

Dans cette réaction complexe, le peroxyde d'hydrogène a deux fonctions : décolorer les pigments en place afin d'éviter les variations de teinture résultant de la couleur initiale du cheveu et déclencher le processus oxydatif.

L'ammoniaque facilite la dissolution des colorants et , par alcalinisation du milieu , favorise l'action décolorante du peroxyde .

Bien que cette technique donne de très bons résultats coloristiques, il est reconnu qu'un traitement répété dans de telles conditions oxydantes alcalines peut dégrader la fibre capillaire , et irriter le cuir chevelu. C'est pourquoi, des recherches sont menées pour trouver une méthode de coloration permanente douce, non agressive pour le cuir chevelu et la fibre capillaire mais qui assure cependant une couverture durable des cheveux .

La technique de coloration non éclaircissante par des enzymes oxydases rendant inutile l'emploi de peroxyde d'hydrogène et d'ammoniaque est une alternative possible.

L'oxydation des polyphénols et des amines aromatiques utilisés pour la coloration permanente peut être catalysée de façon très spécifique par deux groupes d'enzymes: des phénoloxydases (EC 1.14.18.1) ou des peroxydases (EC 1.11.1.7).

L'oxydation activée par les phénoloxydases ne requiert que la présence d'oxygène moléculaire comme co-substrat alors que l'oxydation activée par les peroxydases , comme décrite dans le brevet US 3.957.424, requiert la présence de peroxyde d'hydrogène dans le milieu.

Le choix de phénoloxydases parmi toutes les oxydases connues est donc plus approprié pour catalyser l'oxydation des précurseurs de colorants qui sont leurs substrats spécifiques en présence d'oxygène atmosphérique.

Les phénoloxydases (benzènediol oxygène oxydoréductases ) regroupent deux types d'enzymes:

- les para-diphénoloxydases ou laccases (ancienne classification : EC l.1O.3.2).
- les ortho-diphénoloxydases ou catécholoxydases ou tyrosinases ( ancienne classification: EC l.1O.3.1).

Les laccases catalysent l'oxydation des monophénols, ortho-et paradiphénols, triphénols, para-diamines et de l'acide ascorbique.

Les tyrosinases catalysent l'oxydation des monophénols, des ortho-diphénols, mais pas celle des para-diphénols ni des paradiamines(Mayer et Harel, Phytochem.18, 193-215,l979).

De telles enzymes sont utilisées néanmoins dans des compositions de coloration telle que celle décrite dans le brevet US 2.539.2O2.

Parmi les phénoloxydases, le choix d'une laccase est préférable pour la coloration capillaire, puisque la para-phénylènediamine ou le para-aminophénol, qui sont les précurseurs primaires les plus employés dans la technique de coloration, ne seront pas oxydés ou seulement très lentement en présence de tyrosinase.

Cette technique de coloration activée par des oxydases a fait l'objet de brevets antérieurs.

Le brevet US 3 251 742 décrit une méthode de coloration capillaire activée par des enzymes de type phénolases (tyrosinase ou laccase). Les enzymes sont utilisées soit pour catalyser l'oxydation en présence d'oxygène d'un mélange de composés aromatiques polyhydriques et d'amines, soit pour accélérer leur vitesse d'oxydation en présence d'un agent chimique comme le peroxyde d'hydrogène à pH neutre ou légèrement alcalin (pH 7 à 8,5).

Le brevet FR-A- 2 112 549 reprend cette méthode avec un système d'oxydation qui n'exige pas la présence d'une combinaison des deux types de précurseurs (composés polyhydriques et amines aromatiques) l'un ou l'autre des composés pouvant être utilisés isolément. Ce brevet préconise l'emploi de plusieurs enzymes de type oxydase parmi lesquelles la laccase de Polyporus versicolor, la lactate oxydase de Mycobacterium phlei, la glucose oxydase d'Aspergillus niger, la galactose oxydase de Dactylium

dendroides, la glycolate oxydase du cortex rénal de sanglier, l'aldéhyde oxydase du foie de lapin, la monoamine oxydase du plasma bovin et l'urate oxydase du foie de sanglier.

Le procédé décrit dans ce brevet consiste à mettre en contact les cheveux avec une solution aqueuse renfermant O,O1 à 5OO ppm d'une des oxydases citées et d'environ O,OO1 à 6% en poids de composés aromatiques et ayant une large gamme de pH,de 4 et IO et préférentiellement de 5,5 à 8. Cette solution est exempte de mélanges d'amines aromatiques aromatiques ou de leurs dérivés avec des polyphénols ou leurs dérivés.

La laccase fut découverte en l883 par Yoshida dans le latex de l'Arbre à Laque japonais: Rhus vernicifera (Yoshida, J.Chem. Soc, 472 (l883)). Il semble qu'elle soit présente dans les canaux sécréteurs de tous les membres des Anacardiacées (Joel et al, Phytochem., l7, 796-797, (1984)), dans les pêches et les chataignes et chez de nombreuses espèces de la famille des Podocarpacées.

Chez les champignons, elle est abondamment produite par de nombreux Basidiomycètes qui dégradent la lignine: Collybia velutipes, Fomes annosus, Fomes fomentarius ,Lentinus edodes, Phanerochaete chrysosporium, Pholiota mutabilis, Pleurotus ostreatus,Poria subacida, Sporotrichum pulverulentum, Trametes (= Polyporus) sanguinea, Trametes versicolor. On la trouve chez les Ascomycètes tels qu'Aspergillus nidulans, Neurospora crassa, Podospora anserina et chez les Deutéromycètes tels que Botrytis cinerea et Rhizoctonia praticola (Bollag et Leonowicz , Applied and Environ. Microbiol., 48, 849-854, (l984)).

Ces laccases d'origines diverses forment un groupe relativement hétérogène par la variabilité de leur structure (poids moléculaire, composition) et de leurs propriétés (spécificité par rapport au substrat, pH optimum, point isoélectrique). Cependant toutes les laccases connues catalysent les réactions suivantes:

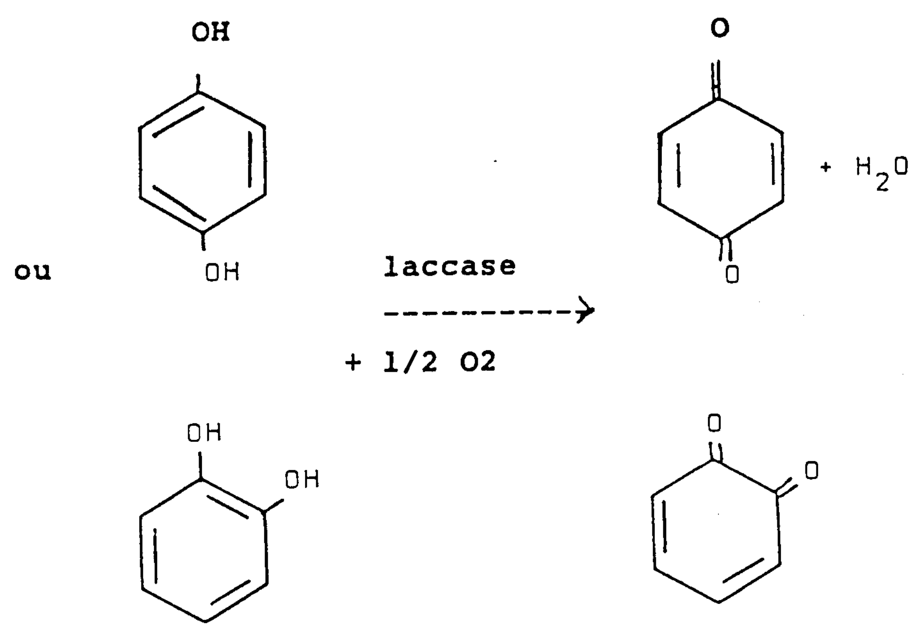


La grande majorité de ces laccases a un pH optimum d'activité acide (< 6,0) pour l'oxydation des phénols et des amines aromatiques à l'exception des laccases de l'Arbre à Laque (Rhus sp.) et de Rhizoctonia praticola qui ont un pH optimum neutre (Reinhammar, B.B.A, 2O5,35-47,(l97O); Bollag et al., Can. J. Microbiol, 25,229-233, (l978)).

Dans le brevet US 3.251.742 , la technique de coloration nécessite le mélange d'un composé aromatique mono ou polyhydrique et d'une amine aromatique . Par ailleurs, les exemples décrits ont été réalisés avec la tyrosinase.

Parmi toutes les oxydases citées dans le brevet FR-A-2 112 549 , la seule laccase indiquée, qui serait l'enzyme la plus spécifique pour catalyser l'oxydation des colorants, est une laccase fongique produite par Trametes (= Polyporus)versicolor. Or cette laccase , qui a été très étudiée, catalyse l'oxydation de phénols et d'amines aromatiques de façon optimale pour des pH compris entre 3,6 et 5,2, avec une activité presque nulle pour des pH supérieurs à 6,O (Benfield, Phytochem.,3,79-88,(l964);Bocks , Phytochem, 6, 777-783,-(1967)). Pour des pH aussi acides, la pénétration des polymères colorés dans la fibre capillaire est très difficile, ce qui rend la coloration moins couvrante et moins résistante aux lavages.

Aucune des compositions décrites dans l'art antérieur ne permet donc une coloration satisfaisante et durable des cheveux sans utiliser de peroxyde d'hydrogène , qui , lors de traitements répétés , dégrade la

fibre capillaire et irrite le cuir chevelu .

La demanderesse s'est donc attachée à la mise en oeuvre d'une composition permettant une coloration non éclaircissante efficace, durable et résistant au lavage des fibres kératiniques et notamment des cheveux, et ne présentant pas les inconvénients précédemment cités , c'est-à-dire globalement n'étant pas agressive pour le cuir chevelu et la fibre capillaire.

La demanderesse a montré de manière surprenante que l'on pouvait colorer des fibres kératiniques et notamment des cheveux , sans les éclaircir à un pH proche de la neutralité, et sans utiliser de peroxyde d'hydrogène , à l'aide d'une composition contenant un enzyme capable de catalyser la formation des polymères colorants, et ayant une activité optimale dans une gamme de pH proche de la neutralité.

De manière encore plus surprenante, la demanderesse a montré que le fait que cette composition ait un pH proche de la neutralité augmente la vitesse initiale de la réaction d'oxydation de certains des précurseurs de colorants.

La présente invention a donc pour objet une composition pour la coloration non éclaircissante des fibres kératiniques et en particulier des cheveux, comprenant notamment un enzyme capable de catalyser la formation des polymères colorants et comprenant aussi des précurseurs de colorants tels que des bases et des coupleurs , dans une solution tamponnée, caractérisée en ce que le pH de ladite composition est proche de la neutralité,et ledit enzyme a une activité optimale dans une gamme de pH comprise entre 6,5 et 8 et ne nécessite pas pour son activité la présence de peroxyde d'hydrogène.

Cette composition permet une pénétration efficace des polymères dans les fibres des cheveux, et permet donc d'obtenir une coloration ton sur ton couvrante et résistante au lavage.

Du fait de la mise en oeuvre d'un enzyme ayant une activité optimale dans une gamme de pH comprise entre 6,5 et 8, la composition peut avoir un pH proche de la neutralité , ce qui permet ainsi d'éviter les inconvénients des compositions décrites dans l'art antérieur , qui ont une certaine agressivité vis-à-vis du cuir chevelu et des fibres capillaires.

Des mesures dynamométriques réalisées sur des mèches de cheveux naturels , colorées par différents procédés de coloration permanente ont permis de mesurer la dégradation provoquée par ces traitements sur la fibre capillaire (altération de ses propriétés élastiques ). Les résultats ont montré qu'une teinture d'oxydation traditionnelle à pH 9,5 renfermant 3% de peroxyde d'hydrogène provoque une dégradation 4 fois supérieure à celle observée avec une coloration enzymatique à pH neutre ( ll% et 3% de dégradation respectivement) .

De plus , la composition selon l'invention offre l'avantage d'éviter l'utilisation de peroxyde d'hydrogène .

En effet , le peroxyde d'hydrogène qui est habituellement utilisé en présence d'ammoniaque provoque à pH alcalin ( $9 < pH < 11$) une décoloration des pigments en place . Ce phénomène apparaît ultérieurement à la racine des cheveux lorsque la fibre s'est allongée de quelques centimètres avec sa pigmentation naturelle . C'est le problème , souvent inesthétique , des "racines ", qui nécessite l'application d'une nouvelle coloration.

Un autre avantage de ce type de composition est son caractère non mutagène.

De manière préférentielle, l'enzyme compris dans la composition est une laccase , en particulier la laccase de Rhizoctonia praticola ou de l'Arbre à Laque (Rhus vernicifera).

Ces deux enzymes , dont l'existence est connue dans l'art antérieur , n'ont jamais été utilisés à la connaissance de la demanderesse dans des compositions pour la coloration des cheveux.

Avantageusement , la laccase de R.praticola est obtenue par fermentation.

La laccase de R.vernicifera peut , quant à elle, être isolée à partir de matériel végétal.

Les bases ou intermédiaires primaires peuvent être des amines aromatiques, des diaminophénols et des aminophénols dont les groupements $NH_2$ et OH sont en position ortho ou para les uns par rapport aux autres . Ils sont responsables de la nuance profonde et peuvent se coupler sur eux-mêmes pour former des pigments très colorés .

Ils peuvent être notamment la para-phénylènediamine (pPD) , l'ortho-aminophénol (oAP), le para-méthylaminophénol (pMAP), le paraaminophénol (pAP), la para-toluylènediamine (pTD) et/ou la N-phényl-para-phénylènediamine (NpPD) .

Les coupleurs ou modificateurs peuvent être des méta-diamines , des métaaminophénols, des polyphénols ou des naphtols. Pris isolément ou en couplage entre eux , ils ne donnent qu'une très faible coloration ; en couplage avec une base , ils modifient la nuance .

Ils peuvent être notamment le méta-aminophénol (mAP) , le pyrocatéchol (PyC) , le pyrogallol(PyG), le résorcinol (R), le l-naphtol (l-N), la méta-phénylènediamine(mPD), le para-aminoorthocrésol (pAOC)-,l'hydroquinone (Hq), le l,5 dihydroxynaphtalène (l.5 DHN) et/ou le 2,7-dihydroxynaphtalène (2.7 DHN) .

L'association bases et coupleurs est choisie en fonction de la couleur désirée.

La formulation globale doit être adaptée au résultat coloristique désiré . On utilise le plus souvent une pluralité d'associations base-coupleur . De bons résultats sont obtenus avec des quantités sensiblement équimolaires pour chaque association base-coupleur prise individuellement.

Les quantités totales de ces molécules sont comprises dans une gamme allant de O,O5% à O,3% en poids de la composition et sont préférentiellement de l'ordre de O,12% environ.

Un autre avantage de la présente composition réside dans le fait qu'il n'est pas obligatoire de mélanger des précurseurs de colorants de type amine avec des précurseurs de type phénol .

Les deux types de précurseurs peuvent être utilisés isolément aussi bien qu'en mélange , ce qui augmente les possibilités coloristiques .

Il est ainsi possible d'obtenir une coloration chatain grâce à un mélange composé uniquement de deux amines aromatiques telles que la p- et la m-phénylènedimaine.

La présente invention a d'autre part pour objet un procédé de coloration des cheveux dans lequel les cheveux sont traités avec la composition précédemment décrite , durant un temps de traitement de lO à 4O minutes et préférentiellement de 2O à 35 minutes . Le traitement est généralement effectué à la température ambiante , mais il peut être accéléré par chauffage doux à 3O°C environ . La température ne doit cependant pas excéder 4O°C .

La description qui suit donne à titre non limitatif des exemples illustratifs de l'invention.

Les figures 1 et 2 représentent l'effet du pH sur l'oxydation respectivement de la pPD et du pAP par des laccases de R.praticola et R.vernicifera (Arbre à Laque). Le pH est mentionné en abscisse, l'ordonnée indiquant le taux en pourcentage de l'activité mesurée par rapport à l'activité maximale des enzymes .

EXEMPLE 1

Procédé de production de la laccase de Rhizoctonia praticola.

Le présent exemple décrit une méthode particulière de culture en fermenteur d'une espèce du genre Rhizoctonia en vue de la production et de la purification de laccase induite.

Ce champignon tellurique produit une phénoloxydase extracellulaire qui a un pH optimum d'activité proche de 7,O alors que la plupart des laccases fongiques ont un pH optimum inférieur à 5,O. Elle est de ce fait très spécifique.

1.Conditions de culture .

Les conditions de culture décrites ici et en particulier les teneurs de certains éléments nutritifs, la nature de l'inoculum, les paramètres d'oxygénation, de température, d'agitation, ainsi que les moments d'apport de l'inducteur et de récolte de la culture ont été définis à la suite de nombreux essais qui ont permis d'optimiser la production de l'enzyme.

Nature de la souche: souche sauvage de l'espèce Rhizoctonia praticola (Vaartaja n°1347= R. solani type AG 4).

Composition du milieu de culture : milieu Czapeck Dow modifié contenant pour un litre:

$NaNO_3$: 3g/$K_2HPO_4$ : lg/KCL: O,5g/$MgSO_4$, 7H2O: O,5 g/ Saccharose: 2Og/ Asparagine: 2,5 g/1ml d'une solution d'oligo-éléments contenant : ($FeSO_4$: lg/$CaCl_2$, $5H_2O$: 2,Og/$CuSO_4$, $5H_2O$: O,l5g/ $ZnSO_4$, $7H_2O$: O,lO g/$H_2O$ qsp lOO ml)/biotine: 25μg/thiamine:5Oμg.

Conditions de fermentation: les paramètres ont été définis avec un réacteur de 7 l contenant 4,5 l utiles, de 42O mm de hauteur et l5O mm de diamètre intérieur:

- temps zéro: ensemencement avec un broyat de mycélium obtenu à partir d'une culture jeune en milieu liquide statique (7O ml d'inoculum pour 4,5 l de milieu).
- pendant 48 heures: agitation à 3OO RPM, température = 28°C, taux d'oxygène dissous = 65%.
- à tO + 48 heures: apport de l'inducteur = 4-méthoxy benzèneamine $2.1O^{-4}$ M dans le milieu;augmentation de l'agitation à 4OO RPM; abaissement de la température à 2O°C et apport d'antimousse (silicones par exemple).
- à tO + 7O à 74 heures: maximum de production de l'enzyme, récolte du milieu et mycélium et filtration pour conserver le milieu qui constitue l'extrait brut.

Cet extrait brut est ensuite purifié pour isoler l'enzyme par les techniques d'ultrafiltration et de chromatographie d'exclusion .

Il subit une ultrafiltration sur membranes de seuil de coupure de lO.OOO daltons puis une séparation par filtration sur Ultrogel AcA 34 (2O OOO- 35O OOO daltons).

2-Caractérisation de l'enzyme:

2-1- pH optimum d'oxydation des phénols et amines aromatiques:

A la différence d'autres laccases d'origine fongique, la laccase de R.praticola a un pH optimum d'activité proche de la neutralité pour oxyder les diphénols ou les p-diamines (pH 6,8 à 7,5). Seule la laccase de l'Arbre à Lacque (Rhus vernicifera) agit de façon optimale à pH neutre pour oxyder les mêmes substrats.

2-2- poids moléculaire (PM):

Il a été déterminé par électrophorèse de l'enzyme sur gel de polyacrylamide contenant du Sodium Dodécyl Sulfate (SDS-PAGE 5-2O%) avec des marqueurs protéiques de PM connus:lactate déshydrogénase 14O000, Albumine Bovine 67 OOO, bêta-glucosidase 36 OOO, Cytochrome C 12 5OO). Le gel coloré par le Bleu de Coomassie a révélé la présence de deux bandes protéiques correspondant à deux iso-enzymes appelés "L1 et L2" de PM égaux à 135 OOO et 155 OOO respectivement.

2-3- point isoélectrique (pI):

Deux techniques de Focalisation Isoélectrique ont indiqué avec précision les pI des 2 isoenzymes séparés.

2-3-1- Focalisation Isoélectrique analytique: Sur Ampholine PAG Plate ( LKB ), pH 3,5-9,5 en présence de marqueurs protéiques de pI connus: Amyloglucosidase 3,5; Ferritine 4,4; Albumine Bovine 4,7; bêta lactoglobuline 5,4: Conalbumine 5,9; Myoglobine cheval 7,3; Ribonucléase 9,45; Cytochrome C IO,65. La révélation au Bleu de Coomassie a montré que les 2 isoenzymes L1 et L2 ont des pI égaux à 4,9 et 4,4 respectivement.

2-3-2-Focalisation isoélectrique préparative:

Sur Ultrodex avec Ampholites pH3-1O a confirmé le pI de la bande L2 possédant la meilleure activité spécifique à 4,4.

CONCLUSION:

La caractérisation de la laccase de R.praticola par son pH optimal d'activité , son poids moléculaire et son point isoélectrique a mis en évidence des propriétés différentes de celles décrites pour d'autres laccases . Ces propriétés spécifiques permettent donc d'identifier aisément la laccase induite de R.praticola produite par fermentation par rapport aux autres laccases (voir tableau ci-après).

**Tableau comparatif des propriétés de différentes laccases:**

| espèce | classe | PM | pI | pH optimum(1) |
|---|---|---|---|---|
| Polyporus versicolor | Basidio mycète | 62000 | ? | 3,8 |
| Rigidoporus lineatus | Basidio mycète | 55000 | 3,5 | 5,6 |
| Agaricus bisporus | Basidio mycète | 102 000 | ? | 5,6 |
| Botrytis cinerea | Asco mycète | 65 000 | 2,5 | 4,7 |
| Rhus vernicifera | Dicoty lédone | 110 000 | 8,5 | 7,0 |
| Rhizoctonia praticola (2) | Deutéro mycète | 78 000 | ? | 7,0 |
| R.prati-cola(3) | Deutéro mycète | 140 000 | 4,4 | 7,0 |

(1) pH optimum d'oxydation de diphénols

(2)culture statique (travaux de J.M. Bollag précédemment cités).

(3) culture en fermenteur.

EXEMPLE 2-

Comparaison de l'activité oxydative des laccases de Rhizoctonia praticola et de Rhus vernicifera en fonction du pH.

Une unité d'activité p-phénylène diamine (upPD) ou une unité d'activité p-aminophénol (upAP) est définie comme la quantité de laccase nécessaire pour provoquer une variation de DO à 525 nm ou à 380 nm d'une unité par minute, à 25°C dans un mélange réactionnel de 2,5 ml de solution tampon phosphate 0,02 M pH 7,0 contenant 0,4 g/l de p-phénylènediamine ou de p-aminophénol.

Les expérimentations ont été effectuées avec des concentrations de précurseurs de colorants d'environ 0,4 g par litre et 0,l unité enzymatique .

Les études effectuées aussi bien sur l'oxydation de la para-phénylène diamine (pPD) que sur l'oxydation du para-aminophénol (pAP) montrent que ces deux laccases ont des activités optimales dans des pH proches de la neutralité, comme on peut le voir sur les figures 1 et 2 résumant les résultats obtenus.

EXEMPLE 3-

Coloration de tissus de laine par la composition selon l'invention .

Les colorations sont obtenues sur des tissus de laine plongés 35 mn à 25 °C dans un mélange réactionnel constitué de 25 ml de solution tampon phosphate O,O2 M, pH 7;,O,5 u de laccase , un mélange équimolaire (environ 2mM) de deux colorants (une base + un coupleur) pour une teneur totale de O,4 g/l.

Les résultats sont résumés dans le tableau I .

L'étude a montré qu'en raison de la grande spécificité des précurseurs de colorants pour l'enzyme (en particulier des molécules aromatiques substituées en ortho et en para), les teneurs en colorants requises pour obtenir une nuance naturelle sont beaucoup plus faibles que celles utilisées en coloration d'oxydation traditionnelle. Par exemple, la teneur maximale en colorants est de 4% environ pour une coloration traditionnelle et de O,2% environ pour une coloration enzymatique selon l'invention. Malgré ces faibles quantités de colorants, les teintures obtenues montrent une résistance aux shampooings tout à fait comparable à celle d'une teinture traditionnelle.

TABLEAU I : Coloration de tissus de laine.

| Coupleur/base | pPD | pAP |
|---|---|---|
| pPD | gris souris légèrement violet | marron beige légèrement rosé |
| pAP | marron beige légèrement rosé | marron beige légèrement rosé |
| oAP | gris vert légèrement jaune | ocre |
| mAP | gris marron légèrement rosé | beige légèrement orangé |
| PyC | gris | marron gris |
| PyG | beige orangé | beige clair |
| R | beige marron | beige clair rosé |
| 1-N | mauve | vieux rose clair |
| mPD | bleu gris | beige marron |
| pAoC | vieux rose | saumon |
| Hq | violet bleu | ocre |
| 1.5 DHN | mauve clair | rose très clair |
| 2.7 DHN | beige | marron beige |

## Revendications

1. Composition pour la coloration non éclaircissante des fibres kératiniques et en particulier des cheveux comprenant notamment un enzyme capable de catalyser la formation des polymères colorants, et

comprenant aussi des précurseurs de colorant tels que des bases et des coupleurs, dans une solution tamponnée , caractérisée en ce que le pH de ladite composition est compris entre 6,5 et 8 et ledit enzyme a une activité optimale dans une gamme de pH comprise entre 6,5 et 8 et ne nécessite pas pour son activité la présence de peroxyde d'hydrogène .

**2.** Composition selon la revendication l, caractérisée en ce que l'enzyme est une laccase, en particulier la laccase de Rhizoctonia praticola ou de Rhus vernicifera.

**3.** Composition selon l'une des revendications l et 2,caractérisée en ce que la laccase de Rhizoctonia praticola est obtenue par fermentation.

**4.** Composition selon l'une des revendications l à 3, caractérisée en ce que les bases sont notamment des amines aromatiques , des diaminophénols et/ou des aminophénols dans lesquels les groupements amines et alcools sont de manière préférentielle en position ortho ou para.

**5.** Composition selon la revendication 4, caractérisée en ce que les bases sont notamment la paraphénylènediamine, l'ortho-aminophénol, le para-méthylaminophénol , le para-aminophénol , la paratoluylènediamine et/ou la N-phényl-paraphénylènediamine.

**6.** Composition selon l'une des revendications l à 5, caractérisée en ce que les coupleurs sont des métadiamines, des métaaminophénols, des polyphénols ou des naphtols, notamment le méta-aminophénol, le pyrocatéchol, le pyrogallol, le résorcinol, le l-naphtol, la métaphénylènediamine, le para-amino-orthocrésol, l'hydroquinone, le l,5-dihydroxynaphtalène, et/ou le 2,7-dihydroxynaphtalène.

**7.** Composition selon l'une des revendications l à 6, caractérisée en ce que le milieu est tamponné à l'aide d'un tampon phosphate.

**8.** Composition selon l'une des revendications l à 7, caractérisée en ce que la quantité totale de précurseur est prise dans la gamme allant de O,O5% à O,3% en poids de la composition et préférentiellement de O,12% .

**9.** Procédé de coloration des cheveux , caractérisé en ce que les cheveux sont traités avec la composition selon l'une des revendications l à 8 .

**10.** Procédé selon la revendication 9 , caractérisé en ce que le temps de traitement est pris dans la gamme allant de lO à 4O minutes et préférentiellement de 2O à 35 minutes, cette durée pouvant être réduite par chauffage doux à une température n'excèdant pas 4O° C.

## Claims

**1.** Composition for the dyeing without lightening of keratinous fibers, and especially hair, comprising, in particular, an enzyme capable of catalyzing the formation of colorant polymers and also comprising dye precursors such as bases and couplers, in a buffered solution, characterized in that the pH of said composition is between 6.5 and 8, and said enzyme has an optimal activity in a pH range of between 6.5 and 8 and does not require the presence of hydrogen peroxide for its activity.

**2.** Composition according to claim 1, characterized in that the enzyme is a laccase, especially Rhizoctonia praticola or Rhus vernicifera laccase.

**3.** Composition according to one of claims 1 and 2, characterized in that the Rhizoctonia praticola laccase is obtained by fermentation.

**4.** Composition according to one of claims 1 to 3, characterized in that the bases are, in particular, aromatic amines, diaminophenols and/or aminophenols in which the amine and alcohol groups are preferably in the ortho or para position.

**5.** Composition according to claim 4, characterized in that the bases are, in particular, paraphenylenediamine, orthoaminophenol, para-methylaminophenol, para-aminophenol, para-toluylenediamine and/or N-phenyl-para-phenylenediamine.

6. Composition according to one of claims 1 to 5, characterized in that the couplers are meta-diamines, meta-aminophenols, polyphenols or naphthols, in particular meta-aminophenol, pyrocatechol, pyrogallol, resorcinol, 1-naphthol, meta-phenylenediamine, para-amino-ortho-cresol, hydroquinone, 1,5-dihydroxynaphthalene and/or 2,7-dihydroxynaphthalene.

7. Composition according to one of claims 1 to 6, characterized in that the medium is buffered using a phosphate buffer.

8. Composition according to one of claims 1 to 7, characterized in that the total amount of precursor is taken within the range extending from 0.05 % to 0.3 % by weight of the composition, and preferably of the order of 0.12 %.

9. Process for dyeing hair, characterized in that hair is treated with the composition according to one of claims 1 to 8.

10. Process according to claim 9, characterized in that the treatment time is taken within the range extending from 10 to 40 minutes, and preferably 20 to 35 minutes, it being possible for this period to be reduced by gentle heating to a temperature not exceeding 40 °C.

**Patentansprüche**

1. Zusammensetzung zur nicht aufhellenden Färbung von Keratinfasern und insbesondere von Haaren, umfassend insbesondere ein Enzym, das die Bildung von Farbstoffpolymeren katalysieren kann, sowie außerdem Farbstoffvorstufen, wie Basen und Kuppler, in einer gepufferten Lösung, dadurch gekennzeichnet, daß der pH-Wert der Zusammensetzung zwischen 6,5 und 8 liegt und das Enzym eine optimale Aktivität in einem pH-Bereich zwischen 6,5 und 8 besitzt und für seine Aktivität nicht der Gegenwart von Wasserstoffperoxid bedarf.

2. Zusammensetzung gemäß Anspruch 1, dadurch gekennzeichnet, daß das Enzym eine Laccase ist, insbesondere die Laccase aus Rhizoctonia praticola oder aus Rhus vernicifera.

3. Zusammensetzung gemäß einem der Ansprüche 1 und 2, dadurch gekennzeichnet, daß die Laccase aus Rhizoctonia praticola durch Fermentation erhalten wird.

4. Zusammensetzung gemäß einem der Ansprüche 1 bis 3, dadurch gekennzeichnet, daß es sich bei den Basen insbesondere um aromatische Amine, Diaminophenole und/oder Aminophenole, bei denen die Amino- und Alkoholgruppen vorzugsweise in ortho- oder para-Stellung zueinander stehen, handelt.

5. Zusammensetzung gemäß Anspruch 4, dadurch gekennzeichnet, daß es sich bei den Basen insbesondere um para-Phenylendiamin, ortho-Aminophenol, para-(Methylamino)phenol, para-Aminophenol, para-Toluylendiamin und/oder N-Phenyl-para-phenylendiamin handelt.

6. Zusammensetzung gemäß einem der Ansprüche 1 bis 5, dadurch gekennzeichnet, daß es sich bei den Kupplern um meta-Diamine, meta-Aminophenole, Polyphenole oder Naphthole, insbesondere um meta-Aminophenol, Brenzkatechin, Pyrogallol, Resorcin, 1-Naphthol, meta-Phenylendiamin, para-Aminoortho-kresol, Hydrochinon, 1,5-Dihydroxynaphthalin und oder 2,7-Dihydroxynaphthalin handelt.

7. Zusammensetzung gemäß einem der Ansprüche 1 bis 6, dadurch gekennzeichnet, daß das Medium mit Hilfe eines Phosphatpuffers gepuffert wird.

8. Zusammensetzung gemäß einem der Ansprüche 1 bis 7, dadurch gekennzeichnet, daß die Gesamtmenge der Vorstufe im Bereich von 0,05 bis 0,3 Gew.-% der Zusammensetzung und vorzugsweise bei 0,12 Gew.-% liegt.

9. Verfahren zum Färben von Haaren, dadurch gekennzeichnet, daß die Haare mit der Zusammensetzung gemäß einem der Ansprüche 1 bis 8 behandelt werden.

**10.** Verfahren gemäß Anspruch 9, dadurch gekennzeichnet, daß die Behandlungszeit im Bereich von 10 bis 40 Minuten und vorzugsweise von 20 bis 35 Minuten liegt, wobei diese Zeitspanne durch leichtes Erwärmen auf eine Temperatur, die 40 °C nicht übersteigt, reduziert werden kann.

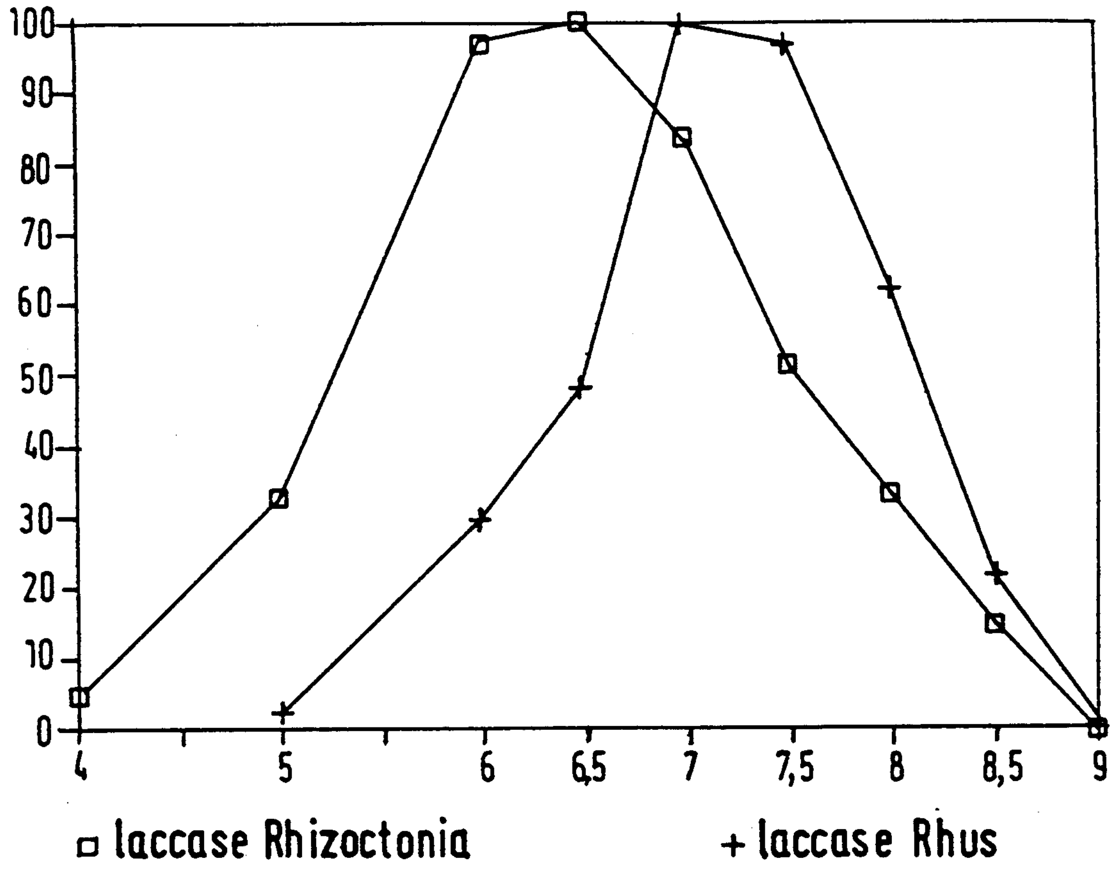
100
90
80
70
60
50
40
30
20
10
0
4
5
6
6,5
7
7,5
8
8,5
9
□ laccase Rhizoctonia
+ laccase Rhus

FIG.1

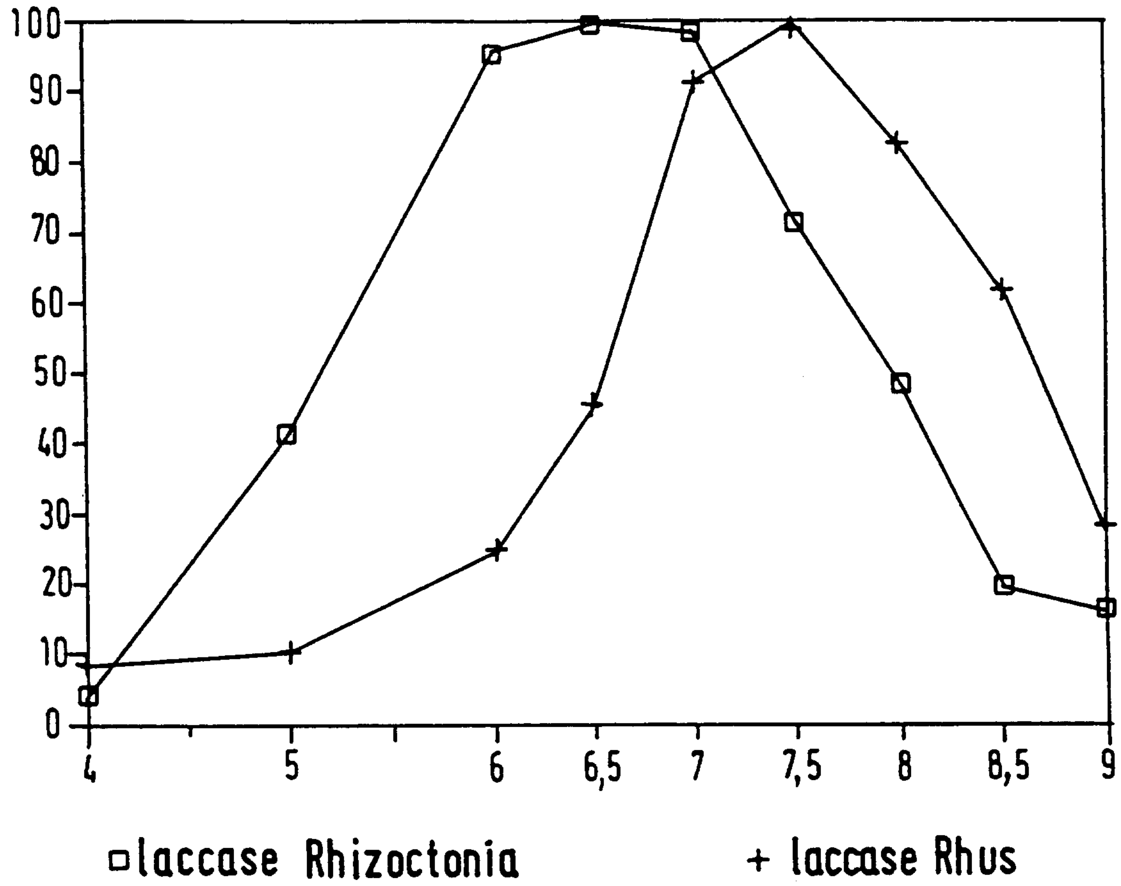
100
90
80
70
60
50
40
30
20
10
0
4
5
6
6,5
7
7,5
8
8,5
9
laccase Rhizoctonia
+ laccase Rhus

FIG.2